(19) Europäisches Patentamt
European Patent Office
Office européen des brevets

(11) Veröffentlichungsnummer: **0 144 640**
**B1**

(12) # EUROPÄISCHE PATENTSCHRIFT

(45) Veröffentlichungstag der Patentschrift: **17.09.86**

(51) Int. Cl.⁴: **C 07 C 121/60**, C 07 C 69/76, C 07 C 25/24, C 07 C 21/24, D 06 L 3/12

(21) Anmeldenummer: **84112434.0**

(22) Anmeldetag: **16.10.84**

(54) **Distyrylverbindungen.**

(30) Priorität: **29.10.83 DE 3339383**

(43) Veröffentlichungstag der Anmeldung: **19.06.85 Patentblatt 85/25**

(45) Bekanntmachung des Hinweises auf die Patenterteilung: **17.09.86 Patentblatt 86/38**

(84) Benannte Vertragsstaaten: **CH DE FR GB LI**

(56) Entgegenhaltungen:
**EP-A-0 032 254**
**DE-A-2 453 357**
**GB-A-1 360 279**

(73) Patentinhaber: **BAYER AG, Konzernverwaltung RP Patentabteilung, D-5090 Leverkusen 1 Bayerwerk (DE)**

(72) Erfinder: **Brinkwerth, Wolfgang, Dr., Am Hang 21, D-5090 Leverkusen 3 (DE)**
Erfinder: **Eckstein, Udo, Dr., Wolfskaul 8, D-5000 Köln 80 (DE)**

**EP 0 144 640 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind Verbindungen der allgemeinen Formel

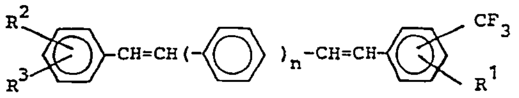 (I)

worin

$R^1$ Wasserstoff, Halogen, Alkyl, Alkoxy, Aminocarbonyl, Cyan, Sulfo, Acyl, Acylamino, Hydroxy, Aryloxy, Aralkoxy, Alkoxycarbonyl oder Acyloxy,

$R^2$ Wasserstoff, Halogen, Aryl, Alkyl, Aralkyl, Hydroxy, Alkoxy, Aralkoxy, Aryloxy, Sulfo, Carboxy, Alkoxycarbonyl, Aryloxycarbonyl, Aralkoxycarbonyl, Aminocarbonyl oder Cyan,

$R^3$ Wasserstoff, Halogen, Alkyl oder $CF_3$ und

n 1, 2 bedeuten und

worin die cyclischen und acyclischen Reste nichtchromophore Substituenten enthalten können.

Die neuen Verbindungen der Formel I sowie deren Gemische sind wertvolle optische Aufheller, die sich dank ihrer geringen Eigenfarbe hervorragend zum Weisstönen von synthetischen Fasermaterialien, insbesondere Polyesterfasern, eignen. Dabei zeichnen sie sich durch eine starke Erniedrigung der Fixiertemperatur und vor allem durch das Fehlen jeglicher Kantenverfärbung der Stoffbahnen im Spannrahmen beim Thermosolfärbeverfahren aus.

In dieser Hinsicht übertreffen sie bei weitem herkömmliche Distyryl-Weisstöner. Selbst den erst neu entwickelten Biscyanstyrylbenzolen gemäss EP-A 23027 und 64303 sind sie deutlich überlegen.

Auch im sogenannten Ausziehverfahren bei Badtemperaturen von 100° C, vorzugsweise 100 bis 130° C (HT-Verfahren), zeigen die neuen Distyrylverbindungen hinsichtlich Weissgrad und Ergiebigkeit ihre überlegenen Eigenschaften.

Die in der Formel I genannten Reste haben folgende bevorzugte Bedeutungen:

Nicht-chromophore Substituenten sind solche, wie sie in der Weisstönerchemie üblich sind, beispielsweise Halogen, Alkyl, Aryl, Aralkyl, Alkoxy, Alkoxycarbonyl, Aminocarbonyl, Cyan, Sulfo, Aminosulfonyl, Acyl, Acylamino, Hydroxy, Aryloxy, Aralkyloxy, Carboxy oder Acyloxy.

Alkyl ist insbesondere $C_1$-$C_4$-Alkyl, das durch Hydroxy, $C_1$-$C_4$-Alkoxy, Cyan, Carboxy, $C_1$-$C_4$-Alkoxycarbonyl, Aminocarbonyl, Chlor oder Brom monosubstituiert sein kann oder Trifluormethyl.

Aryl ist insbesondere gegebenenfalls durch $C_1$-$C_4$-Alkyl, Trifluormethyl, Chlor, Brom, Carboxy, $C_1$-$C_4$-Alkoxycarbonyl oder $C_1$-$C_4$-Alkoxy substituiertes Phenyl.

Aralkyl ist insbesondere Phenyl-$C_1$-$C_4$-Alkyl, das im Phenylkern noch durch Chlor, Methyl oder Methoxy substituiert sein kann.

Alkoxy ist insbesondere $C_1$-$C_4$-Alkoxy.

Acyl ist insbesondere $C_1$-$C_4$-Alkylcarbonyl, $C_1$-$C_4$-Alkoxycarbonyl, $C_1$-$C_4$-Alkylsulfonyl, $C_1$-$C_4$-Alkoxysulfonyl, gegebenenfalls durch Methyl, Methoxy oder Chlor substituiertes Benzoyl, gegebenenfalls durch Methyl, Methoxy oder Chlor substituiertes Benzolsulfonyl, gegebenenfalls durch Methyl, Methoxy oder Chlor substituiertes Phenyl-$C_1$-$C_4$-alkoxycarbonyl oder gegebenenfalls durch Methyl, Methoxy oder Chlor substituiertes Phenoxycarbonyl.

Besonders wertvolle Verbindungen entsprechen der Formel

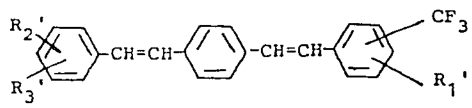 (II)

worin

$R_1'$ Wasserstoff oder Chlor,

$R_2'$ Wasserstoff, Chlor, Phenyl, $C_1$-$C_4$-Alkoxycarbonyl, $C_1$-$C_4$-Alkylsulfonyl, gegebenenfalls durch Chlor, $C_1$-$C_4$-Alkyl, $C_1$-$C_4$-Alkoxy substituiertes Phenylsulfonyl oder Cyano und

$R_3'$ Wasserstoff, Chlor oder $CF_3$ bedeuten.

Ganz besonders bevorzugt sind dabei die unsymmetrischen Typen.

Technischen besonders interessant sind Gemische aus den symmetrischen Verbindungen, den unsymmetrischen Verbindungen und den entsprechenden nicht-$CF_3$-haltigen symmetrischen Verbindungen der Formel I und insbesondere der Formel II, worin

$R_1'$ Wasserstoff oder Chlor,

$R_2'$ $C_1$-$C_4$-Alkoxycarbonyl oder Cyano und

$R_3'$ Wasserstoff oder Chlor bedeuten.

Bevorzugte Mischungen haben folgende Zusammensetzung:

0 bis 35 Gew.-% der symmetrischen Verbindung der Formel

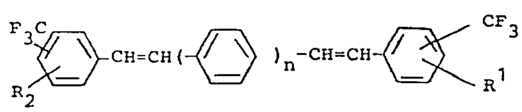 .

($R_1$ = $R_2$)

30 bis 95 Gew.-% der unsymmetrischen Verbindung der Formel

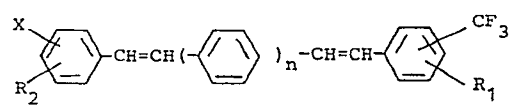

0 bis 35 Gew.-% der Verbindung der Formel

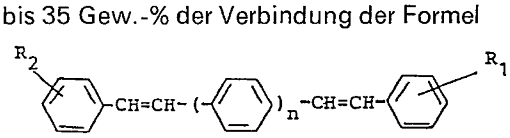

($R_1$ = $R_2$)

sowie entsprechende Mischungen der Formel II.

Die Distyryl-Verbindungen der Formel (I) können nach an sich bekannten Methoden dadurch hergestellt werden, dass man die Verbindung der Formel

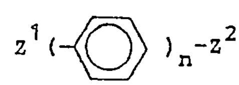 (III)

im gewünschten Verhältnis mit je einer Verbindung der Formeln

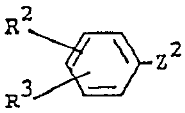 (IVa) bzw. 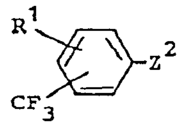 (IVb)

umsetzt, wobei je eines der Symbole $Z_1$ bzw. $Z_2$ eine Formylgruppe und das andere eine Gruppierung der Formel

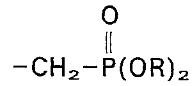 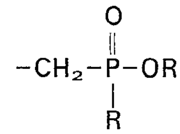

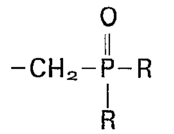 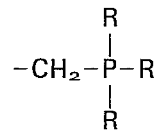

darstellt, worin

R einen $C_1$-$C_4$-Alkyl, einen $C_5$-$C_6$-Cycloalkyl oder einen gegebenenfalls weitersubstituierten Arylrest, vorzugsweise einen Phenylrest bedeutet.

Auf diese Weise können symmetrische und unsymmetrische Verbindungen und Gemische aus symmetrischen und unsymmetrischen Verbindungen hergestellt werden.

Die hierbei als Ausgangsstoffe benötigten Phosphorverbindungen der Formeln (IVa), (IVb) und (III) werden erhalten, indem man Halogenmethylverbindungen, vorzugsweise Chlormethyl- oder Brommethylverbindungen der Formeln

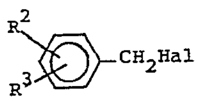 (IVa) bzw.

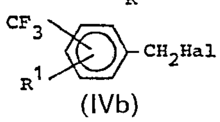 und 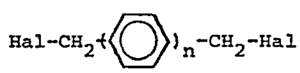

(IVb)

mit Phosphorverbindungen $P(OR)_3$, $R-P(OR)_2$, $RO-P(R)_2$ oder $P(R)_3$ umsetzt, wobei R die angegebene Bedeutung hat. Vorzugsweise bedeutet R an Sauerstoff gebunden $C_1$-$C_4$-Alkyl, an Phosphor gebunden dagegen Phenyl.

Zur Herstellung der Endstoffe kondensiert man die entsprechenden Komponenten in Gegenwart basischer Kondensationsmittel in organischen Lösungsmitteln.

Als Lösungsmittel wählt man vorteilhaft indifferente Lösungsmittel, beispielsweise Kohlenwasserstoffe wie Toluol oder Xylol oder Alkohole wie Methanol, Ethanol, Isopropanol, Butanol, Glykol, Glykolether wie 2-Methoxyethanol, Hexanol, Cyclohexanol, Cyclooctanol, ferner Ether wie Diisopropylether, Dioxan, Tetrahydrofuran; weiterhin Formamide, N-Methylpyrrolidon, Dimethylsulfoxid und Phosphorsäureamide. Bevorzugt sind Dimethylformamid, Dimethylacetamid und Phosphorsäuretris-dialkylamide, wobei Alkyl insbesondere $C_1$-$C_4$-Alkyl ist.

Als Kondensationsmittel kommen stark basische Verbindungen in Betracht wie Alkali- oder Erdalkalimetallhydroxide, Alkali- oder Erdalkaliamide und Alkali- oder Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen.

Man arbeitet bevorzugt im Temperaturbereich von 0 bis 120° C.

Die erfindungsgemässen Verbindungen (I) werden ebenfalls erhalten, wenn man die entsprechenden Aldehydanile in einem dipolar aprotischen Lösungsmittel wie Dimethylformamid in Gegenwart von basischen Kondensationsmitteln mit den entsprechenden Methylverbindungen umsetzt.

Die Verbindungen der Formel (I) zeigen im gelösten oder fein verteilten Zustand eine sehr starke blaue Fluoreszenz. Sie eignen sich einzeln oder als Mischung zum Weisstönen der verschiedensten synthetischen, halbsynthetischen oder natürlichen organischen Materialien.

Besonders bevorzugte Anwendungsgebiete für die erfindungsgemässen Verbindungen und Mischungen (obige Formel I) sind die folgenden: Optisches Aufhellen von Polyester, und zwar sowohl von Polyesterfasern und -gewebe nach dem Auszieh- oder Foulardthermverfahren, als auch von Polyesterspinnmassen, Mischgewebe aus Polyester und Baumwolle oder Wolle werden ebenfalls sehr vorteilhaft mit Hilfe der erfindungsgemässen Verbindungen aufgehellt. Weitere Substrate, die aufgehellt werden können, sind Polyamidfasergewebe, Celluloseacetatgewebe sowie Polystyrol- und Polyvinylchloridmassen. Besonders bevorzugt ist jedoch die Verwendung zum optischen Aufhellen von Polyesterfasern nach dem Auszieh- oder Foulardthermverfahren oder von Polyesterspinnmassen.

Die neuen Weisstöner zeichnen sich dabei durch hohe Farbstärke, gutes Aufbauvermögen und eine geringe Neigung zum Vergrünen aus – selbst bei hohen Wirkstoffkonzentrationen.

Die Menge der erfindungsgemäss zu verwendenden Verbindungen der allgemeinen Formel I, bezogen auf das optisch aufzuhellende Material, kann je nach Einsatzgebiet und gewünschtem Effekt in weiten Grenzen schwanken. Sie kann durch Versuche leicht ermittelt werden und liegt im allgemeinen zwischen etwa 0,01 und etwa 2%.

Die nachfolgenden Beispiele erläutern die Herstellung der erfindungsgemässen Verbindungen und Mischungen sowie deren Verwendung weiter.

*Beispiel 1*

19,5 g (0,1 Mol) 4-Trifluormethyl-benzylchlorid und 23,7 g (0,14 Mol) Triethylphosphit werden bei Rückflusstemperatur 3 Stunden verrührt. Danach wird das überschüssige Triethylphosphit im Vakuum abdestilliert und der Rückstand mit 100 ml wasserfreiem Dimethylformamid versetzt. Zu dieser Lösung gibt man 21 g (0,09 Mol) 4-Formyl-4'-cyano-stilben und tropft innerhalb von 30 Minuten bei 50° C 18 g (0,1 Mol) 30%ige Na-

triummethylat-Lösung zu. Man rührt 4 Stunden bei 50° C und gibt zur Reaktionsmischung 5 g Essigsäure und 50 ml Methanol. Nach Abkühlen auf Raumtemperatur wird der blassgelbe Kristallniederschlag abgesaugt, mit wenig Methanol gewaschen und im Vakuum bei 50° C getrocknet. Man erhält 24,8 g (73,4% der Theorie) blassgelbe, fast farblose Kristalle vom Schmelzpunkt 220 bis 224° C mit der Formel

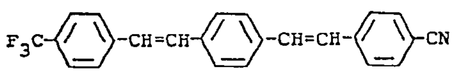 (1)

die aus Toluol umkristallisiert werden können (Schmelzpunkt 225 bis 227° C) UV-Absorption (in DMF) $\lambda_{max}$ = 367 nm $\varepsilon_{max}$ = 65 900

Die Substanz zeigt auf Polyestermaterialien nach dem Thermosolverfahren appliziert hervorragende Weisseffekte mit rötlicher Nuance und insbesondere bei niedrigen Temperaturen ein sehr gutes Fixierverhalten.

*Beispiel 2*

In gleicher Weise, wie in Beispiel 1 beschrieben, erhält man aus 3-Trifluormethylbenzylchlorid 22,3 g (66% der Theorie) fast farblose Kristalle der Verbindung der Formel

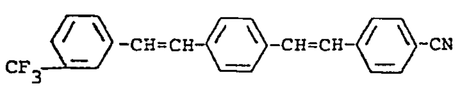 (2)

die aus Toluol umgelöst werden können.
Schmelzpunkt: 132 bis 134° C.
UV-Absorption (in DMF) $\lambda_{max}$ = 365 nm $\varepsilon_{max}$ = 62 900
rotstichige Fluoreszenz in DMF.

*Beispiel 3*

Zu einer Mischung von 3,3 g (0,01 Mol) 2-Chlor-4-trifluormethylbenzylphosphonsäurediethylester und 2,34 g (0,01 Mol) 4-Formyl-4'-cyano-stilben in 25 ml Dimethylformamid tropft man bei 30° C innerhalb 10 Minuten 1,8 g 30%ige Natriummethylat-Lösung. Man lässt 1 Stunde nachrühren, kühlt auf Raumtemperatur ab, gibt wenig Essigsäure hinzu, versetzt mit 10 ml Methanol und filtriert den blassgelben Kristallbrei ab. Man erhält so 2,8 g (68,3% der Theorie) an Rohprodukt der Formel

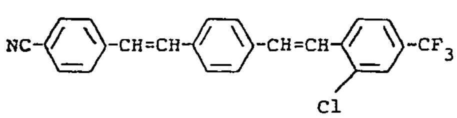 (3)

das durch Umkristallisieren aus Isopropanol gereinigt wird. Schmelzpunkt: 178 bis 180° C.
UV-Absorption ($CHCl_3$) $\lambda_{max}$ = 366 nm $\varepsilon_{max}$ = 59 200
Fluoreszenz (in $CHCl_3$): brillantes blauviolett.

*Beispiel 4*

In analoger Weise, wie in den Beispielen 1 bis 3 beschrieben, werden auch die folgenden wertvollen Verbindungen der Formel

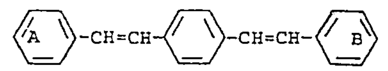 (4)

hergestellt (siehe Tabelle).

*Tabelle I*

| Nr. | A | B | UV-Absorption – Fluoreszenz (in DMF) $\lambda_{max}$ $\varepsilon_{max}$ |
|---|---|---|---|
| 5 | 4-CN | 2-$CF_3$ | 364 nm (62000) neutrales blau |
| 6 | 4-CN | 3-$CF_3$<br>4-Cl | 368 nm (63500) etwas grünstichig blau |
| 7 | 4-CN | 3-$CF_3$<br>2-Cl | 364 nm (60000) blauviolett |
| 8 | 2-CN | 4-$CF_3$ | 360 nm (50250) rotstichig blau |
| 9 | 2-CN | 3-$CF_3$ | 345 nm (40000) stark rotstichig blau |
| 10 | 2-CN | 2-$CF_3$ | 355 nm (46100) blau |
| 11 | 2-CN | 4-$CF_3$<br>2-Cl | 362 nm (58200) intensiv blau |
| 12 | 2-CN | 3-$CF_3$<br>2-Cl | 360 nm (44250) rotstichig blau |
| 13 | 4-$COOC_2H_5$ | 4-$CF_3$ | 363 nm (60270) wenig rotstichig blau |
| 14 | 4-$CF_3$ | 4-$CF_3$ | 360 nm (61700) rotstichig blau |
| 15 | 4-$CF_3$<br>2-Cl | 4-$CF_3$<br>2-Cl | 352 nm (50600) sehr rotstichig blau |
| 16 | 3-$CF_3$<br>4-Cl | 3-$CF_3$<br>4-Cl | 361 nm (55400) blau |

*Beispiel 5*

Man legt 2,7 g (0,02 Mol) 1,4-Diformylbenzol, 5,3 g (0,021 Mol) 4-Cyano-benzylphosphonsäure-diethylester und 6,9 g (0,021 Mol) 2-Chlor-4-trifluormethylbenzylphosphonsäurediethylester in 20 ml Dimethylformamid vor. Zur Mischung werden innerhalb von 30 Minuten bei Raumtemperatur und gutem Rühren 9 g (0,05 Mol) 30%ige Natriummethylat-Lösung getropft. Man rührt 2 Studen bei 40 bis 50° C, versetzt die Reaktionsmischung mit 3 g Essigsäure und filtriert ab. Auf diese Weise erhält man 4,8 g grünlich-weisses Kristallpulver der Zusammensetzung:

72% 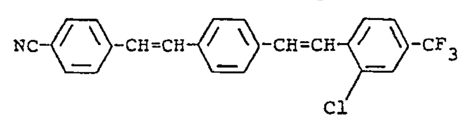

8% 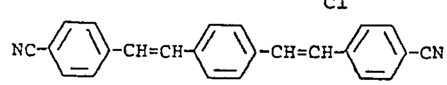 (17)

20% 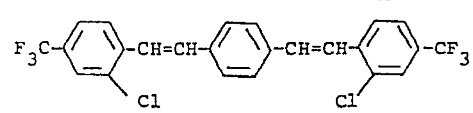

das dünnschichtchromatographisch bestimmt wurde.

UV-Absorption ($CHCl_3$) $\lambda_{max}$ = 362 nm, $\varepsilon_{max}$ = 51 750

Das Gemisch zeigt hervorragende Weisseffekte auf PES-Materialien im Auszieh-HT- und Thermosolverfahren; besonders bei niedrigen Thermosol-Temperaturen ausgiebige Effekte ohne Vergrünung der Kanten.

*Beispiel 6*

Zu einer Mischung aus 12,8 g (0,05 Mol) 4-Cyanobenzylphosphonsäure-diethylester, 14,8 g (0,05 Mol) 2-Trifluormethyl-benzyl-phosphonsäure-diethylester und 6,4 g (0,048 Mol) 1,4-Diformylbenzol in 50 ml Dimethylformamid werden bei Raumtemperatur unter gutem Rühren in 2 Stunden 19,8 g (0,11 Mol) 30%ige Natriummethylat-Lösung zugetropft. Nach 2 Stunden bei 40 bis 50° C wird mit wenig Essigsäure auf pH 4-5 gestellt und der blassgelbe Kristallbrei abfiltriert, mit Methanol gewaschen und getrocknet. Man erhält 15,2 g eines Gemisches der folgenden Zusammensetzung:

88,5% 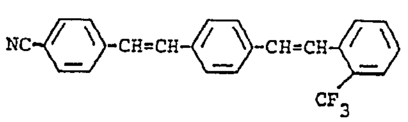

1,5% 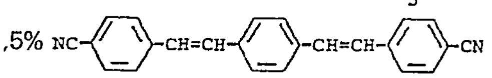 (18)

10% 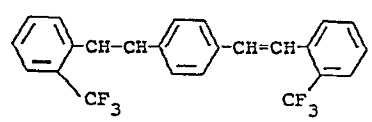

das dünnschichtchromatographisch bestimmt wurde.

UV-Absorption ($CHCl_3$): $\lambda_{max}$ = 359 nm $\varepsilon_{max}$ = 59 000

Das Gemisch zeigt sehr gute rotstichige Weisseffekte auf PES im Auszieh-HT und Thermosolverfahren.

*Beispiel 7*

Auf die gleiche Weise, wie in Beispiel 5 beschrieben, erhält man aus 3,8 g (0,028 Mol) 1,4-Diformylbenzol, 7,6 g (0,03 Mol) 4-Cyanobenzylphosphonsäure-diethylester und 10 g (0,03 Mol) 4-Chlor-3-trifluormethyl-benzylphosphonsäure-diethylester, 12 g hellgelbes Kristallpulver eines Gemisches der Zusammensetzung

62% 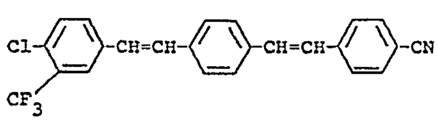

18% 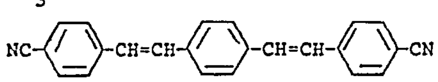 (19)

20% 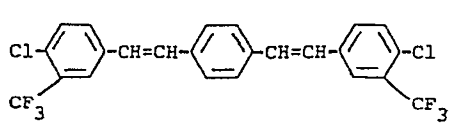

das dünnschichtchromatographisch bestimmt wurde.

UV-Absorption ($CHCl_3$): $\lambda_{max}$ = 362 nm $\varepsilon_{max}$ = 54 200

*Beispiel 8*

Zu einer Lösung von 9,6 g (0,07 Mol) 1,4-Diformylbenzol in 25 ml Dimethylformamid tropft man innerhalb 15 Min. bei 40° C und unter gutem Rühren eine Lösung aus 25 ml Dimethylformamid, 26,7 g (0,09 Mol) 4-Trifluormethyl-benzylphosphonsäure-diethylester und 17,1 g (0,095 Mol) 30%ige Natriummethylat-Lösung. Nach 2-stündigem Nachrühren bei 40 bis 50° C wird dann eine Lösung aus 25 ml Dimethylformamid, 12,7 g (0,05 Mol) 4-Cyano-benzylphosphonsäure-diethylester und 9,9 g (0,055 Mol) 30%ige Natriummethylat-Lösung zugetropft. Man rührt wiederum 2 Stunden bei 40 bis 50° C nach, gibt 4 g Essigsäure zu, entfernt das Lösungsmittel im Wasserstrahlvakuum und versetzt den Rückstand mit 50 ml Methanol. Nach Absaugen und Trocknen erhält man 15,3 g blassgelbes Kristallpulver folgender dünnschichtchromatographisch bestimmter Zusammensetzung:

87% 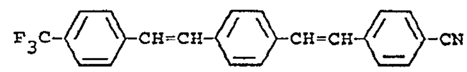

1% 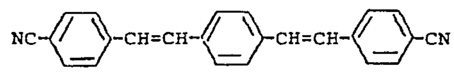 (20)

12% 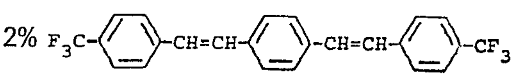

das hervorragend zur Aufhellung von Polyester geeignet ist.

UV-Absorption (in DMF): $\lambda_{max}$ = 366 nm $\varepsilon_{max}$ = 61 300.

*Beispiel 9*

Man verfährt wie im Beispiel 8 und setzt 10,7 g (0,08 Mol) 1,4-Diformylbenzol nacheinander mit 26,7 g (0,09 Mol) 3-Trifluormethyl-benzylphosphonsäure-diethylester und 15,2 g (0,06 Mol) 4-Cyano-benzylphosphonsäure-diethylester um. Man erhält so ein Aufhellergemisch der folgenden Zusammensetzung:

80% 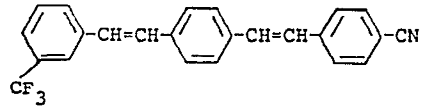

12% 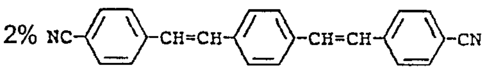 (21)

8% 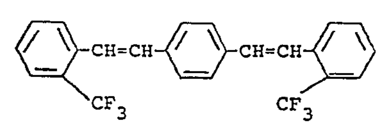

das dünnschichtchromatographisch bestimmt wurde.

UV-Absorption (in DMF) $\lambda_{max}$ = 367 nm $\varepsilon_{max}$ = 60 500

## Patentansprüche

1. Distyrylverbindungen der Formel (I)

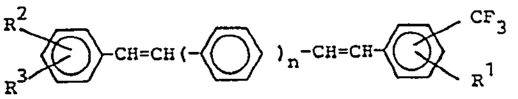 (I)

worin

$R^1$ Wasserstoff, Halogen, Alkyl, Alkoxy, Aminocarbonyl, Cyan, Sulfo, Acyl, Acylamino, Hydroxy, Aryloxy, Aralkoxy, Alkoxycarbonyl oder Acyloxy,

$R^2$ Wasserstoff, Halogen, Aryl, Alkyl, Aralkyl, Hydroxy, Alkoxy, Aralkoxy, Arylkoxy, Sulfo, Carboxy, Alkoxycarbonyl, Aryloxycarbonyl, Aralkoxycarbonyl, Aminocarbonyl oder Cyan,

$R^3$ Wasserstoff, Halogen, Alkyl oder $CF_3$ und

n 1 oder 2 bedeuten und worin

die cyclischen und acyclischen Reste nicht-chromophore Substituenten enthalten können.

2. Distyrylverbindungen gemäss Anspruch 1 der Formel (II)

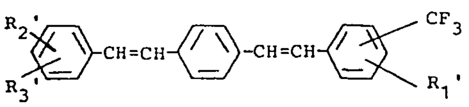 (II)

worin

$R_1'$ Wasserstoff, Chlor

$R_2'$ Wasserstoff, Chlor, Phenyl, $C_1$-$C_4$-Alkoxycarbonyl, $C_1$-$C_4$-Alkylsulfonyl, gegebenenfalls durch Chlor, $C_1$-$C_4$-Alkyl, $C_1$-$C_4$-Alkoxy substituierten Phenylsulfonyl oder Cyano und

$R_3'$ Wasserstoff, Chlor oder $CF_3$ bedeuten.

3. Distyrylverbindungen gemäss Anspruch 2 der Formel (II), worin

$R_1'$ Wasserstoff oder Chlor,

$R_2'$ $C_4$-Alkoxycarbonyl oder Cyano und

$R_3'$ Wasserstoff oder Chlor bedeuten.

4. Mischungen der Verbindungen gemäss Anspruch 1 oder 3, die aus symmetrischen Verbindungen, den unsymmetrischen Verbindungen und den entsprechenden nicht-$CF_3$-haltigen symmetrischen Verbindungen der angegebenen Formel bestehen.

5. Mischung gemäss Anspruch 4, dadurch gekennzeichnet, dass sie aus 0 bis 35 Gew.-% der symmetrischen Verbindungen, 30 bis 95 Gew.-% der unsymmetrischen Verbindungen und 0 bis 35 Gew.-% der entsprechenden nicht $CF_3$-haltigen symmetrischen Verbindung bestehen.

6. Verfahren zur Herstellung von Distyrylverbindungen und Mischungen gemäss Anspruch 1, dadurch gekennzeichnet, dass man die Verbindungen der Formel (III)

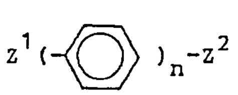 (III)

im gewünschten Verhältnis mit je einer Verbindung der Formel

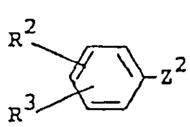 (IVa)

bzw.

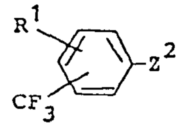 (IVb)

umsetzt, wobei je eines der Symbole $Z_1$ bzw. $Z_2$ eine Formylgruppe und das andere eine Gruppierung der Formel

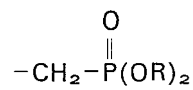

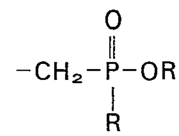

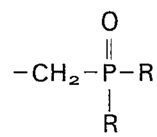

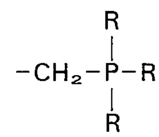

darstellt, worin

R einen $C_1$-$C_4$-Alkyl, einen $C_5$-$C_6$-Cycloalkyl oder einen gebenenfalls weitersubstituierten Arylrest, vorzugsweise einen Phenylrest bedeutet.

7. Verfahren nach Anspruch 6, dadurch gekennzeichnet, dass man die Reaktion in organischen Lösungsmitteln und in Gegenwart basischer Kondensationsmittel umsetzt.

8. Verwendung der Verbindungen der Ansprüche 1 bis 5 als optische Aufheller.

9. Verwendung der Verbindungen der Ansprüche 1 bis 5 zum Weisstönen von Polyesterfasermaterialien nach dem Thermosolverfahren.

## Claims

1. Distyryl compounds of the formula (I)

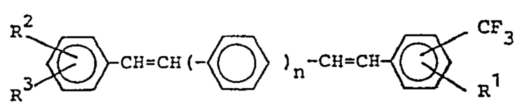 (I)

wherein

$R^1$ denotes hydrogen, halogen, alkyl, alkoxy, aminocarbonyl, cyano, sulpho, acyl, acylamino, hydroxyl, aryloxy, aralkoxy, alkoxycarbonyl or acyloxy,

$R^2$ denotes hydrogen, halogen, aryl, alkyl, aralkyl, hydroxyl, alkoxy, aralkoxy, aryloxy, sulpho, carboxyl, alkoxycarbonyl, aryloxycarbonyl, aralkoxycarbonyl, aminocarbonyl or cyano,

$R^3$ denotes hydrogen, halogen, alkyl or $CF_3$ and

n denotes 1 or 2 and

wherein the cyclic and acyclic radicals may contain non-chromophoric substituents.

2. Distyryl compounds according to Claim 1 of the formula (II)

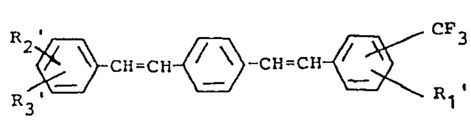 (II)

wherein

$R_1'$ denotes hydrogen or chlorine,

$R_2'$ denotes hydrogen, chlorine, phenyl, $C_1$-$C_4$-alkoxycarbonyl, $C_1$-$C_4$-alkylsulphonyl, optionally chlorine-, $C_1$-$C_4$-alkyl- or $C_1$-$C_4$-alkoxy-substituted phenylsulphonyl or cyano and

$R_3'$ denotes hydrogen, chlorine or $CF_3$.

3. Distyryl compounds according to Claim 2 of the formula (II)

wherein

$R_1'$ denotes hydrogen or chlorine,

$R_2'$ denotes $C_1$-$C_4$-alkoxycarbonyl or cyano and

$R_3'$ denotes hydrogen or chlorine.

.4. Mixtures of the compounds according to Claim 1 or 3 which consist of symmetrical compounds, the asymmetrical compounds and the corresponding non-$CF_3$-containing symmetrical compounds of the indicated formulae.

5. Mixtures according to Claim 4, characterised in that they consist of 0 to 35% by weight of the symmetrical compound, 30 to 95% by weight of the asymmetrical compound and 0 to 35% by weight of the corresponding non-$CF_3$-containing symmetrical compound.

6. Process for preparing distyryl compounds and mixtures according to Claim 1, characterised in that the compounds of the formula (III)

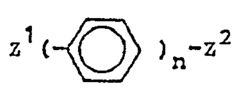 (III)

are reacted in the desired ratio with one compound of each of the formulae

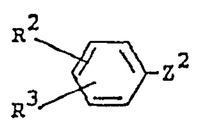 (IVa)

and

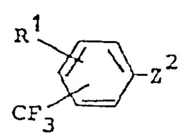 (IVb)

wherein one of the symbols $Z_1$ and $Z_2$ represents a formyl group and the other a grouping of the formula

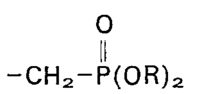

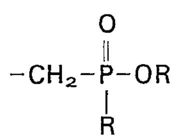

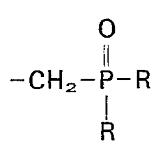

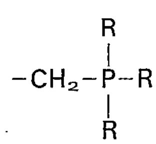

wherein

R denotes a $C_1$-$C_4$-alkyl, a $C_5$-$C_6$-cycloalkyl or an optionally further substituted aryl radical, preferably a phenyl radical.

7. Process according to Claim 6, characterised in that the reaction is carried out in organic solvents and in the presence of basic condensing agents.

8. Use of the compounds of Claims 1 to 5 as fluorescent brighteners.

9. Use of the compounds of Claims 1 to 5 for whitening polyester fibre materials by the thermosol process.

## Revendications

1. Composés distyryliques de formule (I)

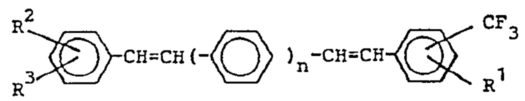 (I)

dans laquelle

$R^1$ désigne l'hydrogène, un halogène, un groupe alkyle, alkoxy, aminocarbonyle, cyano, sulfo, acyle, acylamino, hydroxy, aryloxy, aralkoxy, alkoxycarbonyle ou acyloxy,

$R^2$ désigne l'hydrogène, un halogène, un groupe aryle, alkyle, aralkyle, hydroxy, alkoxy, aralkoxy, aryloxy, sulfo, carboxy, alkoxycarbonyle, aryloxycarbonyle, aralkoxycarbonyle, aminocarbonyle ou cyano.

$R^3$ est l'hydrogène, un halogène, un groupe alkyle ou un groupe $CF_3$ et

n a la valeur 1 ou 2 et

les restes cycliques et acycliques peuvent contenir des substituants non chromophores.

2. Composés distyryliques suivant la revendication 1, de formule (II)

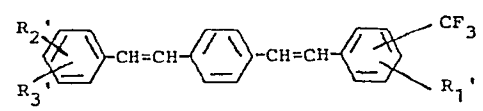 (II)

dans laquelle

$R_1'$ désigne l'hydrogène, le chlore

$R_2'$ désigne l'hydrogène, le chlore, un groupe phényle, (alkoxy en $C_1$ à $C_4$)carbonyle, alkylsulfonyle en $C_1$ à $C_4$, phénylsulfonyle éventuellement substitué par du chlore ou un radical alkyle en $C_1$ à $C_4$ ou alkoxy en $C_1$ à $C_4$, ou le groupe cyano, et

$R_3'$ désigne l'hydrogène, le chlore ou le groupe $CF_3$.

3. Composés distyryliques suivant la revendication 2, de formule (II), dans laquelle

$R_1'$ désigne l'hydrogène ou le chlore,

$R_2'$ est un groupe (alkoxy en $C_1$ à $C_4$)carbonyle ou cyano, et

$R_3'$ est l'hydrogène ou le chlore.

4. Mélanges de composés suivant la revendication 1 ou 3, qui sont constitués par des composés symétriques, les composés asymétriques et les composés symétriques non porteurs du groupe $CF_3$ correspondants des formules indiquées.

5. Mélanges, suivant la revendication 4, caractérisés en ce qu'ils sont formés de 0 à 35% en poids du composé symétrique, 30 à 95% en poids du composé asymétrique et 0 à 35% en poids du composé symétrique non porteur de groupe $CF_3$ correspondant.

6. Procédé de production de composés distyryliques et de mélanges suivant la revendication 1,

caractérisé en ce qu'on fait réagir les composés de formule (III)

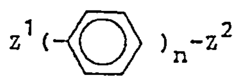 (III)

dans le rapport désiré avec, dans chaque cas, un composé des formules

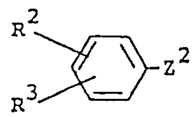 (IVa)

et, respectivement,

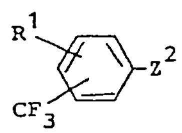 (IVb)

l'un des symboles $Z_1$ et, respectivement, $Z_2$ représentant un groupe formyle et l'autre représentant un groupement de formule

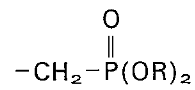

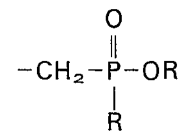

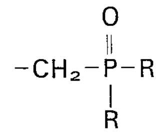

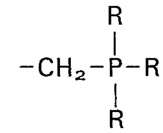

où

R désigne un groupe alkyle en $C_1$ à $C_4$, un groupe cycloalkyle en $C_5$ ou $C_6$ ou un reste aryle portant éventuellement d'autres substituants, de préférence un reste phényle.

7. Procédé suivant la revendication 6, caractérisé en ce qu'on conduit la réaction dans des solvants organiques et en présence d'agents basiques de condensation.

8. Utilisation des composés des revendications 1 à 5 comme azurants optiques.

9. Utilisation des composés des revendications 1 à 5 pour l'azurage de matières en fibres de polyester par le procédé thermosol.